# EUROPEAN PATENT APPLICATION

(11) **EP 4 431 126 A1**
(43) Date of publication of application: **18.09.2024**
(21) Application number: 22890057.7
(22) Date of filing: 07.11.2022
(51) Int. Cl.: A61M 1/18, B01D 63/02, B01D 71/44, B01D 71/52

(54) **HOLLOW FIBER MEMBRANE BLOOD PURIFIER**

(30) Priority: 08.11.2021 JP 2021181881
(71) Applicant: Asahi Kasei Medical Co., Ltd., Tokyo 100-0006 (JP)
(72) Inventor: MINAMI, Miyuki, Tokyo 100-0006 (JP); KITANO, Masaya, Tokyo 100-0006 (JP); NISHIZAWA, Kazuki, Tokyo 100-0006 (JP)
(74) Representative: dompatent von Kreisler Selting Werner - Partnerschaft von Patent- und Rechtsanwälten mbB
(86) International application number: PCT/JP2022/041401
(87) International publication number: WO 2023/080237

(57) **Abstract**

A hollow fiber membrane blood purifier including a hollow fiber membrane and a container to be filled with the hollow fiber membrane, wherein the hollow fiber membrane in the hollow fiber membrane blood purifier contains a fat-soluble material, the proportion of the number of flat fibers in all hollow fibers contained in the hollow fiber membrane blood purifier is 5% or less, and the flat fiber has a flatness ratio calculated from the expression (1): "minor axis of hollow fiber cross section outer diameter/major axis of hollow fiber cross section outer diameter" of 0.5 or less, and the amount of the fat-soluble material immobilized in the hollow fiber membrane is 1 to 500 mg/m².

## Description

### Technical Field

The present invention relates to a hollow fiber membrane blood purifier.

### Background Art

In extracorporeal circulation therapy, a hollow fiber membrane blood purifier that uses a hollow fiber membrane as a selective separation membrane is widely used. For example, the hollow fiber membrane blood purifier is used in hemodialysis as maintenance therapy for a patient with chronic renal failure; in continuous hemofiltration, continuous hemodiafiltration, continuous hemodialysis, or the like as acute blood purification therapy for a patient with serious disease such as acute renal failure or sepsis; and in application of oxygen into blood, separation of plasma, or the like during open heart surgery.

In recent years, hollow fiber membrane blood purifiers in which various characteristics are improved have been proposed. For example, Patent Document 1 proposes a medical hollow fiber blood purifier that is excellent in safety and module assembling properties, is further excellent in the permeability of low-molecular weight materials, and has such high water ultrafiltration rate as to be used in the treatment of chronic renal failure.

### List of Prior Art Documents

### Patent Document

Patent Document 1: Japanese Patent Laid-Open No. 2005-348784

### Summary of Invention

### Problems to be Solved by Invention

In conventional hollow fiber membrane blood purifiers, for example, a technique for applying crimp to the hollow fiber is employed in a spinning step to ensure flowability on the dialysate side. Crimp is applied by applying deformation exceeding the elongation at yield to a part of the circumferential direction of the hollow fiber and the length direction intermittently, and when the degree of deformation is small, the flattening of hollow fibers topically occurs. Thus, in the hollow fiber membrane blood purifier applied with crimp, stress is concentrated on the flat portion of hollow fibers in clinical use, and there is a concern about the occurrence of leakage. To suppress the occurrence of leakage as above, for example, flat fibers are eliminated by process control. However, since such process control is complicated and the process yield is low, there is room for improvement.

In the existing hollow fiber membrane blood purifier containing a fat-soluble material (e.g., vitamin E), the flattening of hollow fibers topically occurs usually in a step of incorporating the fat-soluble material to the hollow fiber membrane (e.g., vitamin E immobilization step). Thus, in the hollow fiber membrane blood purifier containing a fat-soluble material, the flattening of hollow fibers occurs in a higher proportion due to the combination of the flattening of hollow fibers occurred when crimp is applied in the spinning step and the flattening of hollow fibers occurred when the fat-soluble material is incorporated into the hollow fiber membrane.

Examples of the method for incorporating the fat-soluble material into the hollow fiber membrane include a publicly known method (e.g., Japanese Patent Laid-Open No. 2006-296931). In a specific example, a fat-soluble material solution composed of a fat-soluble material and a solvent of the fat-soluble material is prepared first, this is allowed to flow into the hollow fiber lumen part of the hollow fiber membrane to attach the fat-soluble material to the inner surface of the hollow fiber membrane. The excess fat-soluble material solution is then discharged by free fall, air flashing, or the like, and dry gas such as air, nitrogen, or carbon dioxide is introduced to dry the fat-soluble material-covered surface. When the fat-soluble material is incorporated into the hollow fiber membrane, use of a solvent changes the polymer structure of the hollow fiber membrane. In addition, the polymer structure and mechanical strength of the hollow fiber membrane, and further, the hollow fiber shape may vary due to the discharge conditions of the fat-soluble material solution and the introduction conditions of dry gas. In the process, when a pressure exceeding the pressure resistance strength is applied from the outside of the hollow fiber membrane to the hollow fiber membrane, for example, during the discharge of the fat-soluble material or the introduction of dry gas, flat fibers are generated. Moreover, when uneven attachment of the fat-soluble material to the hollow fiber membrane, uneven discharge upon discharge of the fat-soluble material, or the like occurs due to the concentration of the fat-soluble material solution, the fat-soluble material solution is topically discharged from a portion where the fat-soluble material is less attached, dry gas is concentrated on a portion having high gas permeability, and flattening easily occurs starting from the portion. As described above, the mechanism of the generation of flat fibers in the step of incorporating a fat-soluble material is different from the mechanism of the generation of flat fibers occurring upon applying crimp during spinning.

As the method for suppressing the occurrence of the flattening of hollow fibers, for example, application of crimp to the hollow fiber membrane in a special manner has been proposed. For example, in the hollow fiber blood purifier described in Patent Document 1, when crimp is applied to the hollow fiber membrane, the flattening of hollow fibers is suppressed by applying deformation exceeding the elongation at yield to a part of the circumferential direction of the hollow fiber and the length direction intermittently after the hollow fiber membrane structure is substantially immobilized, at a temperature of 50°C or less to apply partial elongation, thereby substantially immobilizing the crimp state. However, the flatness proportion of the hollow fiber described in Patent Document 1 is a proportion in a state where no fat-soluble material is immobilized, and the hollow fiber membrane blood purifier containing a fat-soluble material is not examined in any way. As mentioned above, in the hollow fiber membrane blood purifier containing a fat-soluble material, flat fibers are generated in a higher proportion by a generation mechanism that is different from that of the flat fibers generated upon applying crimp, so that a technique for suppressing the generation of flat fibers in a higher level is desired.

Examples of the method for hemodialysis include hemodialysis (HD) and hemodiafiltration (HDF). Above all, as a method effective for the treatment of dialysis complications, hemodiafiltration therapy accounts for about half of the methods recently used for dialysis treatment. Hemodiafiltration is a therapy in which substitution fluid is injected, and the pressure during treatment is more likely to rise than in hemodialysis. Thus, to suppress the pressure rise during treatment, the hollow fiber inner diameter of a hemodiafilter is designed to be larger than that of a hemodialyzer, in many cases. In addition, since hemodiafiltration is a therapy for removing unnecessary materials by filtration, the transmembrane pressure applied to the hollow fiber membrane is higher than that in hemodialysis. Thus, the risk of leakage during treatment starting from the flattening of the hollow fiber membrane tends to be higher in hemodiafiltration. Consequently, a technique for suppressing the generation of flat fibers at a higher level is desired in the hollow fiber membrane blood purifier used in hemodiafiltration.

However, when a fat-soluble material is immobilized on a hollow fiber membrane having a large inner diameter using a known technique for immobilizing a fat-soluble material, flat fibers are generated in a higher proportion due to the reduction in compressive strength and pressure resistance strength of the hollow fiber resulting from a large inner diameter of the hollow fiber, and there is a concern about use in hemodiafiltration with a high leakage risk, which is problematic.

In the hollow fiber membrane blood purifier containing a fat-soluble material, in particular, in the hollow fiber membrane having a large inner diameter to be used in hemodiafiltration therapy, a technique for suppressing the generation of flat fibers that allows the occurrence of leakage in clinical use to be suppressed for a long time is desired to achieve higher treatment safety, which can not be not solved by the known technique for immobilizing a fat-soluble material.

### Means for Solving Problems

The present inventors have intensively studied to solve the above problems, and as a result, found that the occurrence of leakage can be suppressed for a long time by controlling the number of flat fibers in all hollow fibers to a specific range in the hollow fiber membrane blood purifier containing a fat-soluble material, and thus accomplished the present invention.

That is, the present invention is as follows.
[1] A hollow fiber membrane blood purifier comprising a hollow fiber membrane and a container to be filled with the hollow fiber membrane, wherein
   the hollow fiber membrane in the hollow fiber membrane blood purifier contains a fat-soluble material,
   a proportion of the number of flat fibers in all hollow fibers contained in the hollow fiber membrane blood purifier is 5% or less, and the flat fibers have a flatness ratio calculated from the expression (1) of 0.5 or less, and
   an amount of the fat-soluble material immobilized in the hollow fiber membrane is 1 to 500 mg/m²,
   minor axis of hollow fiber cross section outer diameter/major axis of hollow fiber cross section outer diameter ... expression (1).
[2] The hollow fiber membrane blood purifier according to [1], wherein the flat fibers having a flatness ratio calculated from the expression (1) of 0.5 or less include flat fibers having a flatness ratio calculated from the expression (1) of 0.2 or more and 0.5 or less.
[3] The hollow fiber membrane blood purifier according to [1], wherein the flat fibers having a flatness ratio calculated from the expression (1) of 0.5 or less include flat fibers having a flatness ratio calculated from the expression (1) of 0.3 or more and 0.5 or less.
[4] The hollow fiber membrane blood purifier according to any of [1] to [3], wherein a material constituting the hollow fiber membrane contains at least one selected from the group consisting of a hydrophilic polymer and a hydrophobic polymer.
[5] The hollow fiber membrane blood purifier according to [4], wherein the hydrophilic polymer contains at least one selected from the group consisting of polyvinylpyrrolidone (PVP) and polyethylene glycol (PEG).
[6] The hollow fiber membrane blood purifier according to any of [1] to [5], wherein the fat-soluble material contains at least one selected from the group consisting of vitamin A, vitamin D, vitamin E, and vitamin K.
[7] The hollow fiber membrane blood purifier according to any of [1] to [6], wherein the amount of the fat-soluble material immobilized in the hollow fiber membrane is 1 to 300 mg/m².
[8] The hollow fiber membrane blood purifier according to any of [1] to [6], wherein the amount of the fat-soluble material immobilized in the hollow fiber membrane is 1 to 10 mg/m².
[9] The hollow fiber membrane blood purifier according to any of [1] to [8], wherein an inner diameter of the hollow fiber membrane is 170 µm or more and 250 µm or less.
[10] The hollow fiber membrane blood purifier according to any of [1] to [8], wherein an inner diameter of the hollow fiber membrane is 180 µm or more and 250 µm or less.
[11] The hollow fiber membrane blood purifier according to any of [1] to [8], wherein an inner diameter of the hollow fiber membrane is 180 µm or more and 220 µm or less.
[12] The hollow fiber membrane blood purifier according to any of [1] to [11], wherein a membrane thickness of the hollow fiber membrane is 10 µm or more and 50 µm or less.

### Advantages of Invention

The present invention can suppress the occurrence of leakage for a long time in the hollow fiber membrane blood purifier containing a fat-soluble material.

### Mode for Carrying Out Invention

To solve the aforementioned problems, for example, vitamin E has been immobilized on a hollow fiber membrane having a large inner diameter, in particular, an inner diameter of 180 µm or more and 250 µm or less by using a known vitamin E immobilization technique, and the flatness ratio of the generated flat fibers has been measured. As a result, it has been newly found that there are almost no flat fibers in which the lumen part of the hollow fiber is completely blocked, most flat fibers have a flatness ratio of 0.2 to 0.5, and in particular, a large part of flat fibers have a flatness ratio of 0.3 to 0.5. The flatness ratio of the flat fiber is calculated by the following expression (1).

Minor axis of hollow fiber cross section outer diameter/major axis of hollow fiber cross section outer diameter ... expression (1)

Hereinafter, the embodiment for implementing the present invention (hereinafter, referred to as "the present embodiment") will be described in detail. However, the present invention is not limited to the following embodiment, and can be variously modified within the scope of the gist thereof and implemented.

### <Hollow Fiber Membrane Blood Purifier>

The hollow fiber membrane blood purifier of the present embodiment is a hollow fiber membrane blood purifier including a hollow fiber membrane and a container to be filled with the hollow fiber membrane, wherein the hollow fiber membrane in the hollow fiber membrane blood purifier contains a fat-soluble material, the proportion of the number of flat fibers in all hollow fibers contained in the hollow fiber membrane blood purifier is 5% or less, and the flat fiber has a flatness ratio calculated from the expression (1) of 0.5 or less, and
the amount of the fat-soluble material immobilized in the hollow fiber membrane is 1 to 500 mg/m².

With such a configuration, the hollow fiber membrane blood purifier of the present embodiment can suppress the occurrence of leakage for a long time.

In the present embodiment, the flat fiber refers to a hollow fiber having a flatness ratio determined by the following expression (1) of 0.5 or less. Flatness ratio = minor axis of hollow fiber outer diameter/major axis of hollow fiber outer diameter

Meanwhile, in the present embodiment, hollow fibers in which the shape of the hollow fiber membrane is irregular shape and the hollow fiber outer diameter cannot be measured, or non-circular or non-oval hollow fibers in which the major axis and the minor axis are not clear refer to the irregular fiber, and are not included in the definition of the flat fiber in the present embodiment.

Herein, the flatness ratio of a hollow fiber cross section on which the flatness ratio is lowest in a hollow fiber is employed as the flatness ratio. Although it is not limited to the method described below, in the measurement of the hollow fiber outer diameter, for example, a flat portion of a hollow fiber is specified with a microwatcher or the like, the flat portion is cut using a razor or the like so as not to deform the hollow fiber shape, and the dimension of the cross section is measured. The dimension of the portion at which the outer diameter of the hollow fiber cross section is longest is employed as the major axis and the dimension of the portion at which it is shortest is employed as the minor axis, and the flatness ratio is then calculated by the above expression (1).

In the measurement, 500 fibers among all hollow fibers contained in the hollow fiber membrane blood purifier are randomly sampled, and the number of flat fibers in these 500 fibers is measured.

In the hollow fiber membrane blood purifier of the present embodiment, the proportion of the number of flat fibers in all hollow fibers is preferably 0 to 5%, more preferably 0 to 4%, and still more preferably 0 to 3%. When the proportion of the number of flat fibers in all hollow fibers is within the range, there is a tendency that the hollow fiber membrane blood purifier of the present embodiment can suppress the occurrence of leakage for a long time.

In the present embodiment, the method for controlling the proportion of the number of flat fibers in all hollow fibers to the aforementioned range is not particularly limited, and examples thereof include the method for carrying out a step of immobilizing the fat-soluble material described below.

The hollow fiber membrane blood purifier of the present embodiment is used in, for example, extracorporeal circulation blood purification therapy such as hemodialysis, hemofiltration, hemodiafiltration, blood component fraction, application of oxygen, and separation of plasma, without particular limitation.

The hollow fiber membrane blood purifier of the present embodiment is preferably used as a hemodialyzer, a hemofilter, a hemodiafilter, or the like, and is more preferably used as a continuous hemodialyzer, a continuous hemofilter, and a continuous hemodiafilter which are continuous applications thereof. The detailed specifications such as the dimension and fractionation properties of the hollow fiber membrane are determined according to each application.

From the viewpoint of permeability, crimp is preferably applied to the hollow fiber membrane used in the hollow fiber membrane blood purifier of the present embodiment. The method for applying crimp is not particularly limited, and a publicly known method can be used.

### <Fat-Soluble Material>

In the hollow fiber membrane blood purifier of the present embodiment, the hollow fiber membrane contains a fat-soluble material.

In the present embodiment, the fat-soluble material refers to a material that is generally hardly soluble in water and is soluble in alcohols and fats and oils, and a natural or synthetic material having low toxicity can be used. Specific examples of the fat-soluble material include, but are not particularly limited to, cholesterol; vegetable oils such as castor oil, lemon oil, and shea butter; animal oils such as fish oil; fatty acids such as sucrose fatty acid ester and polyglycerol fatty acid ester; isoprenoid; hydrocarbons having a large number of carbon atoms; and silicone oils. In addition, vitamin A, vitamin D, vitamin E, and vitamin K which are lipophilic vitamins, and ubiquinone and the like are also preferably used.

Above all, the fat-soluble material preferably contains at least one selected from the group consisting of vitamin A, vitamin D, vitamin E, and vitamin K. Among these, vitamin E is preferable from the viewpoint that excess intake thereof does not induce disorder. The fat-soluble material may be used singly or as a mixture of two or more kinds.

Examples of vitamin E include, but are not particularly limited to, α-tocopherol, β-tocopherol, γ-tocopherol, δ-tocopherol, and mixtures thereof. Examples of vitamin E derivatives include, but are not particularly limited to, α-tocopherol acetate, α-tocopherol nicotinate, and a mixture thereof. Above all, α-tocopherol is preferable since it is excellent in various physiological effects such as an in vivo antioxidant effect, a biomembrane stabilizing effect, and a platelet aggregation inhibition effect, and has a high effect of suppressing oxidative stress.

### <Amount of Fat-Soluble Material Immobilized>

The amount of the fat-soluble material immobilized in the hollow fiber membrane is preferably 1 to 500 mg/m², more preferably 1 to 300 mg/m², and still more preferably 1 to 10 mg/m². When the amount of the fat-soluble material immobilized in the hollow fiber membrane is the lower limit value or more, sufficient antioxidant performance can be obtained, and when it is the upper limit value or less, hemocompatibility is excellent. Above all, in the hollow fiber having the amount of the fat-soluble material immobilized of 1 to 10 mg/m², uneven immobilization of the fat-soluble material in the hollow fiber membrane can be reduced and the generation of flat fibers can be further suppressed in the step of immobilizing the fat-soluble material, in addition to the above effects.

In the present embodiment, "the amount of the fat-soluble material immobilized in the hollow fiber membrane" refers to the amount of the fat-soluble material that is bonded to, attached to, adsorbed to, or covers the entire hollow fiber membrane, and the amount of the fat-soluble material present on the entire hollow fiber membrane can be quantitatively determined by, for example, the amount of the fat-soluble material extracted with a solvent without breaking or dissolving the hollow fiber membrane, as described below.

One example of the method for measuring the amount of the fat-soluble material in the hollow fiber membrane will be described.

First, the hollow fiber membrane blood purifier into which the hollow fiber membrane is incorporated is disassembled, and the hollow fiber membrane is collected, washed with water, and then subjected to drying treatment. Subsequently, a surfactant or an organic solvent that dissolves the fat-soluble material, for example, 1% by mass of an aqueous polyethylene glycol-t-octylphenyl ether solution or ethanol is added to the precisely weighed hollow fiber membrane after drying, and the mixture is stirred and extracted. The membrane area of the hollow fiber membrane is the nominal hollow fiber membrane area provided by a manufacturer (the hollow fiber membrane area described in a label), or the inner surface area calculated from the product of the average inner diameter (diameter), circumference ratio, number, and effective length of the hollow fiber membrane. The effective length refers to the hollow fiber length having permeability, excluding the urethane sealing part and the like from the entire length of the hollow fiber membrane.

The quantitative manipulation is carried out by, for example, liquid chromatograph, and the concentration of the fat-soluble material in an extraction liquid is calculated by using a calibration curve obtained from the peak area of the standard solution of the fat-soluble material.

The amount of the fat-soluble material (mg/m²) in the hollow fiber membrane can be determined from the obtained concentration of the fat-soluble material and the extracted membrane area of the hollow fiber membrane by taking the extraction efficiency as 100%.

The liquid chromatograph described as an example can be conducted as follows. A column (ODP-506E packed column for HPLC manufactured by Shodex Asahipak) is attached to a high performance liquid chromatography apparatus (pump: JASCO PU-1580, detector: SHIMADZU RID-6A, Autoinjector: SHIMADZU SIL-6B, data processing: Tosoh GPC-8020, column oven: GL Sciences556), methanol for high-performance liquid chromatography as a mobile phase is made to flow, for example, at a flow rate of 1 mL/min at a column temperature of 40°C, and the concentration of the fat-soluble material is determined from the absorption peak area at a wavelength of 295 nm using a UV detector.

### <Hydrophilic Polymer and Hydrophobic Polymer>

The material constituting the hollow fiber membrane preferably contains at least one selected from the group consisting of a hydrophilic polymer and a hydrophobic polymer. When the hollow fiber membrane contains such a material, the hollow fiber membrane tends to be a membrane having high physical strength and being excellent in biocompatibility.

In particular, the hollow fiber membrane preferably contains a hydrophilic polymer at least on a separation function surface. By containing a hydrophilic polymer, the hollow fiber membrane tends to have good biocompatibility.

The hydrophilic polymer is not particularly limited as long as it is a polymer having a high affinity for water, and examples thereof include polymers having a solubility parameter δ (cal/cm3)1/2 of 10 or more and polymers having a hydroxy group.

The solubility parameter δ is an index described in, for example, "Polymer Data Handbook, Basic Edition" edited by The Society of Polymer Science, Baifukan Co., Ltd., published on January 30, 1986, first edition, pp. 591 to 593, and a high solubility parameter indicates high hydrophilicity, and a low solubility parameter indicates high hydrophobicity.

Examples of the polymer having a solubility parameter δ (cal/cm3)1/2 of 10 or more include, but are not particularly limited to, polyhydroxyethyl methacrylate (δ = 10.00), cellulose diacetate (δ = 11.35), and polyacrylonitrile (δ = 12.35). The value described as δ is described as one example.

Examples of the polymer having a hydroxy group include, but are not particularly limited to, polyhydroxyalkyl methacrylates such as polyhydroxyethyl methacrylate, polyhydroxypropyl methacrylate, and polyhydroxybutyl methacrylate, and sodium salts of polysaccharides such as sodium alginate, sodium hyaluronate, and sodium heparin. Polyhydroxyalkyl methacrylate is a synthetic polymer obtained by (co)polymerizing hydroxyalkyl methacrylate as a monomer unit and is a compound having a hydroxy group on a side chain.

Examples of the hydrophilic polymer include, but are not particularly limited to, polyvinylpyrrolidone (hereinafter, also referred to as "PVP"), polyethylene glycol (hereinafter, also referred to as "PEG"), polyvinyl alcohol (hereinafter, also referred to as "PVA"), and polypropylene glycol.

These hydrophilic polymers may be used singly or as a mixture of two or more kinds thereof.

Above all, at least one selected from the group consisting of polyvinylpyrrolidone (PVP) and polyethylene glycol (PEG) is preferably contained as the hydrophilic polymer. When the hollow fiber membrane contains such a hydrophilic polymer, the hollow fiber membrane tends to have good biocompatibility.

Examples of the hydrophobic polymer include, but are not particularly limited to, polysulfone resins such as polysulfone (PS), polyethersulfone (PES), and polyarylethersulfone (PAES); cellulose resins such as regenerated cellulose, cellulose acetate, and cellulose triacetate (CTA); polyacrylonitrile (PAN), polyvinylidene fluoride (PVDF), polymethyl methacrylate, and an ethylene vinyl alcohol copolymer. Above all, a polysulfone resin is preferable.

The polysulfone resin is a sulfonyl (-SO2-) group-containing synthetic polymer and is excellent in heat resistance and chemical resistance. Examples of the polysulfone resin include, but are not particularly limited to, polyphenylenesulfone, polysulfone, polyarylethersulfone, polyethersulfone, and copolymers thereof. The polysulfone resin may be used singly or as a mixture of two or more kinds thereof.

Above all, from the viewpoint of controlling fractionation properties, a polysulfone polymer represented by the following formula (1) or the following formula (2) is preferable.

(-Ar-SO2-Ar-O-Ar-C(CH3)2-Ar-O-)n (1)

(-Ar-SO2-Ar-O-)n (2)

In the formula (1) and the formula (2), Ar represents a benzene ring, and n represents a repetition of the monomer unit. Polysulfone represented by the formula (1) is commercially available, for example, under the name of "Udel(trademark)" from Solvay and under the name of "Ultrason(trademark)" from BASF, and polyethersulfone represented by the formula (2) is commercially available under the name of "SUMIKAEXCEL(trademark)" from Sumitomo Chemical Co., Ltd. There are several types depending on the degree of polymerization and the like, and these can be appropriately utilized.

### <Antioxidant>

In the present embodiment, the hollow fiber membrane is preferably sterilized in an aqueous antioxidant solution, from the viewpoint of hemocompatibility, and this is preferable since the oxidation of, in particular, the fat-soluble material in the hollow fiber membrane in a sterilization step is suppressed.

The antioxidant is not particularly limited, as long as it is an atom, molecule, or ion having properties of easily imparting electrons to other molecules and the like, and examples thereof include an ultraviolet absorber and a light stabilizer, a metal deactivator, an ozone deterioration inhibitor, an amine antioxidant, a phenolic antioxidant, a sulfur antioxidant, and a phosphorous antioxidant.

Examples of the ultraviolet absorber and the light stabilizer include, but are not particularly limited to, phenyl salicylate, monoglycol salicylate, p-tert-butylphenyl salicylate, 2-hydroxy-4-methoxybenzophenone, 2-hydroxy-4-octoxybenzophenone, and 2(2'-hydroxy-5'-methylphenyl)benzotriazole.

Examples of the metal deactivator include, but are not particularly limited to, N-salicyloyl-N'-aldehydehydrazine, N-salicyloyl-N'-acetylhydrazine, and N,N'-diphenyl oxamide.

Examples of the ozone deterioration inhibitor include, but are not particularly limited to, 6-ethoxy-2,2,4-trimethyl-1,2-dihydroquinoline and N-phenyl-N'-isopropyl-p-phenylenediamine.

Examples of the amine antioxidant include, but are not particularly limited to, phenyl-β-naphthylamine, α-naphthylamine, and phenothiazine.

Examples of the phenolic antioxidant include, but are not particularly limited to, 2,6-di-tert-butyl-p-cresol, 2,6-di-tert-butylphenol, and 2,4-di-methyl-6-tert-butyl-phenol.

Examples of the sulfur antioxidant include, but are not particularly limited to, dilauryl thiodipropionate, lauryl stearyl thiodipropionate, dilauryl sulfide, sodium hydrogen bisulfite, sodium pyrosulfite, sodium sulfite, sodium hydrogen pyrosulfite, sodium hydrosulfide, and acetone sodium hydrogen bisulfite.

Examples of the phosphorous antioxidant include, but are not particularly limited to, triphenyl phosphite and tridodecyl phosphite.

The antioxidant is not particularly limited, and for example, L-ascorbic acid, cysteine, thioglycerol, or isopropyl citrate may be used.

The antioxidant may be used singly or as a mixture of two or more kinds thereof.

Above all, sodium hydrogen bisulfite, sodium pyrosulfite, sodium sulfite, or sodium hydrogen pyrosulfite is preferable from the viewpoint of handling properties.

When the hollow fiber membrane is sterilized in an aqueous antioxidant solution, it is suitable that an antioxidant be dissolved in water and then the hollow fiber membrane be irradiated with radiation in a state of being wetted by the aqueous solution.

The concentration of the antioxidant in the aqueous antioxidant solution is preferably 50 ppm or more and 2000 ppm or less. A pH adjusting agent such as sodium carbonate; sodium chloride, glycerin or the like may coexist in the aqueous antioxidant solution, in addition to the antioxidant.

### <Method for Producing Hollow Fiber Membrane>

The hollow fiber membrane used in the present embodiment can be produced by utilizing a known dry and wet(dry-wet) film forming technique. Examples of the method include, but are not particularly limited to, a method having a spinning step of performing a dry spinning method to obtain a membrane intermediate material containing a hydrophilic polymer and a hydrophobic polymer, and an immobilization step of immobilizing a fat-soluble material on the membrane intermediate material.

In the hollow fiber membrane used in the present embodiment, it is not necessary to use both the hydrophilic polymer and the hydrophobic polymer, and the hollow fiber membrane may be a single material membrane.

In the step of producing the hollow fiber membrane, examples of the method include, but are not limited to, a method in which a tube-in-orifice spinneret is used and a spinning dope and a hollow inner liquid for coagulating the spinning dope are simultaneously discharged from the orifice of the spinneret and the tube, respectively, into the air.

As the hollow inner liquid, water or a coagulation liquid mainly made of water can be used, and the composition thereof may be determined according to the permeability of the hollow fiber membrane of interest. Typically, a mixed solution of a solvent used in the spinning dope and water is suitably used.

The spinning dope discharged from the spinneret with the hollow inner liquid is made to run on an air gap part, introduced into a coagulation bath mainly made of water installed on a lower part of the spinneret, immersed therein, thereby completing coagulation. After passing through a washing step and the like, the hollow fiber membrane in a wet state is wound with a winding machine to obtain a hollow fiber membrane bundle, which is then subjected to drying treatment. Alternatively, after passing through the above washing step, the hollow fiber membrane may be dried in a drying machine to obtain a hollow fiber membrane bundle.

Specific examples of the method for producing the hollow fiber membrane used in the present embodiment include, but are not particularly limited to, a method for carrying out a usual membrane formation step by using a membrane-forming spinning dope containing a polysulfone resin and polyvinylpyrrolidone.

The membrane-forming spinning dope can be adjusted by, for example, dissolving a polysulfone resin and polyvinylpyrrolidone in a solvent.

Examples of such a solvent include, but are not particularly limited to, dimethylacetamide, dimethylsulfoxide, N-methyl-2-pyrrolidone, dimethylformamide, sulfolane, and dioxane.

The solvent may be used singly or as a mixed solvent of two or more kinds thereof.

The concentration of the polysulfone resin in the membrane-forming spinning dope is not particularly limited, as long as it is in the range of concentrations at which the membrane can be formed and the resulting membrane has the performance as a permeation membrane, and is preferably 5% by mass or more and 35% by mass or less, and more preferably 10% by mass or more and 30% by mass or less when the resin composition is taken as 100% by mass. When high water ultrafiltration rate is achieved, a lower polysulfone resin concentration is preferable, and the concentration is still more preferably 10% by mass or more and 25% by mass or less.

The polyvinylpyrrolidone concentration in the membrane-forming spinning dope is adjusted so that the mixing ratio of polyvinylpyrrolidone to the polysulfone resin is preferably 27% by mass or less, more preferably 18% by mass or more and 27% by mass or less, and still more preferably 20% by mass or more and 27% by mass or less.

By setting the mixing ratio of polyvinylpyrrolidone to the polysulfone resin to 27% by mass or less, the amount of polyvinylpyrrolidone eluted can be suppressed. Suitably, by setting the mixing ratio to 18% by mass or more, the polyvinylpyrrolidone concentration of the separation function surface can be controlled to a suitable range, the effect of suppressing the protein adsorption can be enhanced, and the hemocompatibility is excellent.

Using the membrane-forming spinning dope, a hollow fiber membrane is formed by a method usually used. For example, using a tube-in-orifice spinneret, a membrane-forming spinning dope and a hollow inner liquid for coagulating the membrane-forming spinning dope are simultaneously discharged from the orifice of the spinneret and the tube, respectively, into the air. As the hollow inner liquid, water or a liquid mainly made of water can be used. Typically, a mixed solution of a solvent used in the membrane-forming spinning dope and water is suitably used as the hollow inner liquid. For example, 20% by mass or more and 70% by mass or less of an aqueous dimethylacetamide solution or the like can be used.

By adjusting the amount of the membrane-forming spinning dope discharged and the amount of the hollow inner liquid discharged, the inner diameter and membrane thickness of the hollow fiber membrane can be adjusted to a desired value.

The inner diameter of the hollow fiber membrane is generally only required to be, for example, 170 µm or more and 250 µm or less in blood treatment applications, preferably 180 µm or more and 250 µm or less in hemodiafiltration applications, and more preferably 180 µm or more and 220 µm or less. From the viewpoint of the diffusion and removal efficiency of low-molecular weight materials by the mass transfer resistance as the permeation membrane, the membrane thickness of the hollow fiber membrane is preferably 50 µm or less. From the viewpoint of strength, the membrane thickness of the hollow fiber membrane is preferably 10 µm or more.

In the present embodiment, the inner diameter and membrane thickness of the hollow fiber membrane can be measured by the method described in Examples described below.

The membrane-forming spinning dope discharged from the spinneret together with the hollow inner liquid is made to run on an air gap part, introduced into a coagulation bath mainly made of water installed on a lower part of the spinneret, and then immersed therein for a certain time, thereby completing coagulation. In this case, a draft represented by the ratio of the linear discharge velocity of a membrane-forming spinning dope to the take-over speed thereof is preferably 1 or less.

The air gap refers to a space between the spinneret and the coagulation bath, and in the membrane-forming spinning dope, coagulation is initiated from the inner surface side by a poor solvent component such as water in the hollow inner liquid simultaneously discharged from the spinneret. The draft is preferably 1 or less, and more preferably 0.95 or less since a smooth hollow fiber membrane surface is formed and the hollow fiber membrane structure is stable upon initiation of coagulation.

Then, after the solvent remaining on the hollow fiber membrane is removed by washing with hot water or the like, the hollow fiber membrane is put in a drying chamber to remove the moisture in the hollow fiber.

### <Method for Producing Hollow Fiber Membrane Blood Purifier>

The hollow fiber membrane blood purifier is assembled based on the hollow fiber membrane obtained through the above steps. First, a cylindrical container having two nozzles in the vicinity of both end parts of the side surface is filled with the hollow fiber membrane, and both end parts thereof are embedded with a urethane resin. Then, the cured urethane portions are cut and processed so that the end parts at which the hollow fiber membrane is open are formed. A header cap having a nozzle for letting a liquid such as blood or dialysate in (out) is loaded to the both end parts, whereby the shape of the hollow fiber membrane blood purifier is assembled.

### <Step of Immobilizing Fat-Soluble Material on Hollow Fiber Membrane>

In the present embodiment, the proportion of the number of flat fibers in all hollow fibers can be controlled to the aforementioned range by carrying out the following coating method as the step of immobilizing the fat-soluble material on the hollow fiber membrane. The coating method is a method for attaching the fat-soluble material on the hollow fiber membrane surface by flowing a fat-soluble material solution into the hollow part on the inner surface side of the hollow fiber membrane.

In the coating method, the fat-soluble material is immobilized on the formed hollow fiber membrane, and then the hollow fiber membrane blood purifier may be assembled, or the fat-soluble material may be immobilized by allowing the coating solution to flow after assembling or in the middle stage of assembling the hollow fiber membrane blood purifier.

The concentration of the fat-soluble material in the coating solution is preferably 0.004% by mass or more and 30% by mass or less, more preferably 0.01% by mass or more and 20% by mass or less, and still more preferably 0.01% by mass or more and 10% by mass or less, when the total amount of the coating solution is taken as 100% by mass. When the concentration of the fat-soluble material in the coating solution is the lower limit or more, it is possible to control the amount of the fat-soluble material immobilized that allows antioxidant potential to be maintained, and when it is the upper limit or less, uneven immobilization of the fat-soluble material can be reduced.

The step of immobilizing the fat-soluble material includes three steps: a liquid-infusing step, a dewatering step, and drying step.

The liquid-infusing step is a step of infusing a coating solution obtained by dissolving a fat-soluble material (e.g., vitamin E) in an organic solvent (e.g., 2-propanol) to the inside of the hollow fiber. In this case, the amount of the coating solution is preferably set to 500 mL or more.

In the dewatering step, excess coating solution remaining on the hollow fiber inner surface, the hollow fiber outer surface, and the hollow fiber membrane thickness part is discharged to the outside of the module by allowing compressed air to pass through. The compressed air is fed from either the inside or the outside of the hollow fiber or from both of them upon dewatering, and at this time, the pressure difference between the inside and the outside of the hollow fiber (inside - outside) is -0.1 to 1.2 MPa, and preferably 0 to 0.5 MPa, that is, the pressure difference between the inside and the outside of the hollow fiber is made small. To suppress the crush of the hollow fiber due to the pressure from the outside of the hollow fiber, the pressure difference is preferably -0.1 MPa or more which is below the compressive strength of the hollow fiber, and to suppress the burst of the hollow fiber due to the pressure from the inside of the hollow fiber, the pressure difference is preferably 1.2 MPa or less which is below the pressure resistance strength of the hollow fiber. Further, to suppress the coating solution from leaking out to the outside of the hollow fiber due to high pressure of the inside of the hollow fiber, the pressure difference is more preferably 0.5 MPa or less.

In the drying step, air or an inert gas such as nitrogen is allowed to pass through the module after dewatering to remove the solvent in the coating solution. The temperature of the inert gas is preferably 20 to 80°C. In addition, an inert gas is sent to the inside and the outside of the hollow fiber in the drying step. The air flow rate of the inside of the hollow fiber at this time is preferably 300 to 600 mL/min or more, and the air flow rate of the outside of the hollow fiber is preferably 50 to 100 mL/min or more.

In the present embodiment, by carrying out the step of immobilizing a fat-soluble material on a hollow fiber membrane, the generation of flat fibers in the step can be suppressed and the proportion of the number of flat fibers in all hollow fibers can be easily and efficiently controlled to the aforementioned range. In this case, the amount of the fat-soluble material immobilized is 1 to 500 mg/m².

Flat fibers are generated when the pressure difference between the inside and the outside of the hollow fiber during dewatering exceeds the compressive strength or pressure resistance strength of the hollow fiber, and the concentration of the fat-soluble material of the coating solution is also considered to affect the generation of flat fibers. When the concentration of the fat-soluble material of the coating solution is high, the amount of the fat-soluble material attached to the surface of the entire hollow fiber membrane increases, so that uneven attachment of the fat-soluble material easily occurs. Consequently, it is considered that dewatering topically occurs during dewatering from a portion where the attached fat-soluble material is little, compressed air is concentrated on a portion having high gas permeability, and flattening easily occurs starting from the portion. Thus, by adjusting the concentration of the fat-soluble material of the coating solution to, for example, 0.004 to 0.04% by mass and by controlling the amount of the fat-soluble material immobilized to, for example, 1 to 10 mg/m², the generation rate of flat fibers in the step can be suppressed at a lower level.

### <Wetting Step of Hollow Fiber Membrane>

As for the assembled hollow fiber membrane blood purifier, the hollow fiber membrane is preferably wetted with an aqueous solution before sterilization from the viewpoint of protecting the hollow fiber membrane, and more preferably sterilized in an aqueous antioxidant solution. Examples of the method for wetting the hollow fiber membrane with an aqueous solution include a method for filling a container filled with the hollow fiber membrane with an aqueous solution, and a method for filling a container with an aqueous solution and then draining the solution with air flashing or the like. Above all, a method of filling the module with sodium pyrosulfite and/or an aqueous sodium carbonate solution in is preferable.

### <Sterilization Step of Blood Treatment Device>

The hollow fiber membrane blood purifier is preferably subjected to sterilization treatment. Examples of the sterilization method include radiation sterilization, electron beam sterilization, high pressure steam sterilization, and ethylene oxide gas (EOG) sterilization.

Since the hollow fiber membrane containing the fat-soluble material has a risk of causing a hollow fiber failure due to extreme heating, radiation sterilization is preferable. In radiation sterilization, an electron beam, a gamma ray (γ ray), an X-ray, or the like may be used. The irradiation dose of radiation is, in the case of a γ ray or an electron beam, preferably 15 kGy or more and 50 kGy or less, and more preferably 20 kGy or more and 40 kGy or less.

### Examples

Hereinafter, the present invention will be specifically described with reference to Examples and Comparative Examples, but the present invention is not limited by these Examples. The measurement methods used in Examples are as follows.

### <Internal Diameter and Membrane Thickness of Hollow Fiber Membrane>

The inner diameter and membrane thickness of the hollow fiber membrane were measured as follows.

First, a hollow fiber membrane was cut perpendicular to the axial direction with a razor. Cutting was performed so that the hollow fiber membrane was not crushed and deformed upon cutting. Internal diameters and membrane thicknesses at arbitrary three points in the cross sections of the cut hollow fiber membrane were measured with a microscope, and average values thereof were determined as the inner diameter and the membrane thickness of the hollow fiber membrane.

### <Proportion of Number of Flat Fibers in All Hollow Fibers>

The proportion of the number of flat fibers in all hollow fibers contained in the hollow fiber membrane blood purifier was calculated as follows.

First, the hollow fibers contained in the hollow fiber membrane blood purifier were observed one by one while rotating in a radial direction, and a portion where the hollow fiber is flattest was specified. The flat portion was cut with a razor perpendicular to the longitudinal direction of the hollow fiber so that the shape is not deformed, and the dimension of the cross section was measured with a digital microscope VHX-6000 (manufactured by KEYENCE CORPORATION) at an observation magnification of 100 times. Herein, the dimension of the portion where the hollow fiber outer diameter was longest was employed as the major axis of the hollow fiber outer diameter and the dimension of the portion where the hollow fiber outer diameter was shortest was employed as the minor axis of the hollow fiber outer diameter, and the flatness ratio of each hollow fiber was calculated according to the following expression (1). Flatness ratio = minor axis of hollow fiber outer diameter/major axis of hollow fiber outer diameter

A hollow fiber having a flatness ratio determined by the above expression (1) of 0.5 or less was determined as the flat fiber. Based on the calculated flatness ratio of each hollow fiber, the proportion (%) of the number of flat fibers in all hollow fibers was determined. Hollow fibers in which the shape of the hollow fiber membrane was irregular shape and the hollow fiber outer diameter was not successfully measured, or non-circular or non-oval hollow fibers in which the major axis and the minor axis were not clear were determined as the irregular fiber, and the proportion (%) of the number of irregular fibers in all hollow fibers was also determined.

In the measurement, using a hollow fiber membrane blood purifier, 500 fibers among all hollow fibers contained in the hollow fiber membrane blood purifier were randomly sampled, and the number of flat fibers in these 500 fibers was measured.

### <Amount of Fat-Soluble Material Immobilized in Hollow Fiber Membrane>

The hollow fiber membrane blood purifier was washed with water, dried, and then disassembled, and the hollow fiber membrane was collected and cut into about 4 cm. Washing with water was carried out at 5 L/min for 2 minutes. Drying was carried out with the air at room temperature at 60 L/min or more for 6 hours or more. 381 mL of ethanol per membrane area (m²) of the hollow fiber membrane blood purifier was added to the collected hollow fiber, and the fat-soluble material was extracted while applying ultrasonic vibration at room temperature for 60 minutes. The quantitative manipulation was carried out by liquid chromatograph, and the amount of the fat-soluble material in the extraction liquid was determined by using the calibration curve obtained from the peak area of the standard solution of the fat-soluble material.

A column (Shodex Asahipak, ODP-506E packed column for HPLC) was attached to a high performance liquid chromatography apparatus (pump: JASCO PU-1580, detector: SHIMADZU RID-6A, Autoinjector: SHIMADZU SIL-6B, data processing: Tosoh GPC-8020, column oven: GL Sciences556), methanol for high-performance liquid chromatography as a mobile phase was made to flow at a flow rate of 1 mL/min at a column temperature of 40°C, and the concentration of the fat-soluble material was determined from the absorption peak area of an ultraviolet part. The amount of the fat-soluble material immobilized in the hollow fiber membrane (mg/m²) was determined from this concentration by taking the extraction efficiency as 100%.

The amount of the fat-soluble material partially oxidized by the sterilization treatment was also included in the amount of the fat-soluble material per 1 m² of the hollow fiber membrane surface. To determine the amount of the fat-soluble material partially oxidized by the sterilization treatment, radiation of 50 kGy was applied in advance in the air to the fat-soluble material to be used to prepare the calibration curve, and the absorption peak of the partially oxidized fat-soluble material determined in advance was included in the peak groups to be used for area calculation, and added.

### <Measurement of Leakage Time>

With respect to the hollow fiber membrane blood purifier, a pulse test for evaluating the leakage resistance properties of the hollow fiber was carried out as follows by mimicking blood pulsation in clinical use.

Water at 37°C was allowed to flow at a flow rate of 800 mL/min into the hollow fiber membrane blood purifier from the dialysate inlet side, and discharged from the dialysate outlet side. At this time, 0.3 MPa of compressed air was allowed to flow into the hollow fiber membrane blood purifier from the blood inlet side for 1 second, and then, the compressed air flowed into the blood side was released simultaneously when the inflow of the compressed air was stopped for 1 second. This operation was repeatedly and continuously carried out. The time (leakage time) from the inlet of the compressed air into the hollow fiber membrane blood purifier until leakage occurs through the hollow fiber and the leakage of the compressed air (bubbles) occurs on the dialysate side was measured. The case where the leakage time was 300 min or more was evaluated that the occurrence of leakage was successfully suppressed for a long time. The leakage time of a hollow fiber membrane blood purifier immobilized with a fat-soluble material, which was already used in clinical use, was measured by the above method and found to be about 250 minutes. Since the average treatment time of typical dialysis is 3 to 5 hours per treatment, it was determined that the leakage time of 300 min or more in the present leakage test allows the hollow fiber leakage during clinical use to be suppressed.

### [Example 1]

A membrane forming dope composed of 17 parts by mass of polysulfone (P-1700 manufactured by Solvay Japan Co., Ltd.), 4 parts by mass of polyvinylpyrrolidone (hereinafter, also referred to as "PVP") (K-90 manufactured by BASF), and 79 parts by mass of dimethylacetamide (hereinafter, also referred to as "DMAc") (special grade reagent, Kishida Chemical Co., Ltd.) was prepared. A 58% by mass aqueous DMAc solution was used as the hollow inner liquid, and discharged from a spinneret having a slit width of 50 µm. At this time, the temperature of the membrane forming dope upon discharge was 40°C. The discharged dope passed through a fall part covered with a hood was immersed in a coagulation bath at 60°C made of water for coagulation. At this time, the air gap length was set to 400 mm and the spinning rate was set to 34 m/min. The resultant was washed with water and dried to obtain a blood treatment membrane. The amount of the membrane forming dope and hollow inner liquid discharged was adjusted so that the membrane thickness after drying was 43 µm and the inner diameter was 200 µm.

Then, a cylindrical container having two nozzles for letting liquid in and out was filled with a bundle of 13000 hollow fiber membranes wound after drying, both end parts thereof were embedded with a urethane resin. Then, the cured urethane portions were cut, and the both end parts were processed into end parts at which the hollow fiber membrane was open. A header cap having a nozzle for letting blood in (out) was loaded to the both end parts, whereby the shape of the hollow fiber membrane blood purifier having a membrane area of 2.2 m² was assembled.

Then, by carrying out the following liquid-infusing step, dewatering step, and drying step, vitamin E was immobilized on the hollow fiber membrane as the fat-soluble material.

### (Liquid-infusing step)

500 mL of a coating solution (vitamin E coating solution) obtained by dissolving 0.9% by mass of vitamin E (α-tocopherol (Wako Pure Chemical Industries, Ltd. special grade)) in an aqueous solution of 57% by mass of isopropanol was infused from an inlet nozzle on the blood side (hereinafter, also referred to as the "B side") of the hollow fiber membrane blood purifier at a temperature of 24°C to the inner surface side of the hollow fiber membrane to bring vitamin E into contact with the hollow fiber membrane. At this time, the coating solution was infused to the inside of the hollow fiber with the port on the dialysate side (hereinafter, also referred to as the "D side") of the hollow fiber membrane blood purifier closed.

### (Dewatering step)

Then, the hollow fiber membrane blood purifier was set so that the pressure difference between the inside (B side) and the outside (D side) of the hollow fiber (inside - outside) was -0.05 MPa, and dewatering was carried out by feeding compressed air from the B side and the D side.

### (Drying step)

Thereafter, air was fed to the B side at 300 mL/min and to the D side at 50 mL/min, and drying was performed until the solvent in the hollow fiber membrane blood purifier was removed.

Through the above liquid-infusing step, dewatering step, and drying step, vitamin E was immobilized on the hollow fiber membrane. The amount of vitamin E immobilized in the hollow fiber membrane was 235 mg/m².

Then, the wetting step was carried out as follows. First, the hollow fiber membrane blood purifier was washed with water. Thereafter, the blood side flow path (inner surface side) and the filtrate side flow path (outer surface side) of the hollow fiber membrane blood purifier were filled with an aqueous solution containing 0.06% by mass of sodium pyrosulfite as an antioxidant and further containing 0.03% by mass of sodium carbonate for adjusting pH.

After the wetting step, the hollow fiber membrane blood purifier was irradiated with γ ray at 25 kGy for sterilization with each nozzle sealed, thereby obtaining the hollow fiber membrane blood purifier.

The proportion of the number of flat fibers in all hollow fibers contained in the obtained hollow fiber membrane blood purifier was calculated to be 3%. More specifically, in all hollow fibers, flat fibers having a flatness ratio of 0 to less than 0.2 were 0%, flat fibers having a flatness ratio of 0.2 to less than 0.3 were 0.4%, flat fibers having a flatness ratio of 0.3 to less than 0.4 were 1.7%, and flat fibers having a flatness ratio of 0.4 to 0.5 were 0.9%. Note that the proportion of the number of irregular fibers in all hollow fibers was 0.5%.

### [Example 2]

The hollow fiber membrane blood purifier of Example 2 was obtained in the same manner as in Example 1, except that the amount of the coating solution in the liquid-infusing step for immobilizing the fat-soluble material was changed to 800 mL, as shown in Table 1. In this case, the amount of vitamin E immobilized in the hollow fiber membrane was 245 mg/m². The proportion of the number of flat fibers in all hollow fibers was calculated to be 4% by the above method. More specifically, in all hollow fibers, flat fibers having a flatness ratio of 0 to less than 0.2 were 0%, flat fibers having a flatness ratio of 0.2 to less than 0.3 were 0.8%, flat fibers having a flatness ratio of 0.3 to less than 0.4 were 2.0%, and flat fibers having a flatness ratio of 0.4 to 0.5 were 1.2%. Note that the proportion of the number of irregular fibers in all hollow fibers was 0.3%.

### [Example 3]

The hollow fiber membrane blood purifier of Example 3 was obtained in the same manner as in Example 1, except that the pressure difference between the inside (B side) and the outside (D side) of the hollow fiber (inside - outside) in the dewatering step for immobilizing the fat-soluble material was changed to 0.05 MPa, as shown in Table 1. In this case, the amount of vitamin E immobilized in the hollow fiber membrane was 237 mg/m². The proportion of the number of flat fibers in all hollow fibers was calculated to be 1% by the above method. More specifically, in all hollow fibers, flat fibers having a flatness ratio of 0 to less than 0.2 were 0%, flat fibers having a flatness ratio of 0.2 to less than 0.3 were 0.2%, flat fibers having a flatness ratio of 0.3 to less than 0.4 were 0.4%, and flat fibers having a flatness ratio of 0.4 to 0.5 were 0.4%. Note that the proportion of the number of irregular fibers in all hollow fibers was 0%.

### [Example 4]

The hollow fiber membrane blood purifier of Example 4 was obtained in the same manner as in Example 1, except that vitamin E dissolved in the vitamin E coating solution was changed to 0.8% by mass in the liquid-infusing step, and the air flow rate on the B side was changed to 600 mL/min and the air flow rate on the D side was changed to 100 mL/min in the drying step, as shown in Table 1. In this case, the amount of vitamin E immobilized in the hollow fiber membrane was 205 mg/m². The proportion of the number of flat fibers in all hollow fibers was calculated to be 3% by the above method. More specifically, in all hollow fibers, flat fibers having a flatness ratio of 0 to less than 0.2 were 0%, flat fibers having a flatness ratio of 0.2 to less than 0.3 were 0.9%, flat fibers having a flatness ratio of 0.3 to less than 0.4 were 1.1%, and flat fibers having a flatness ratio of 0.4 to 0.5 were 1.0%. Note that the proportion of the number of irregular fibers in all hollow fibers was 0.2%.

### [Example 5]

The hollow fiber membrane blood purifier of Example 5 was obtained in the same manner as in Example 1, except that vitamin E dissolved in the vitamin E coating solution was changed to 0.8% by mass in the liquid-infusing step, and the pressure difference between the inside (B side) and the outside (D side) of the hollow fiber (inside - outside) in the dewatering step was changed to 0 MPa, as shown in Table 1. In this case, the amount of vitamin E immobilized in the hollow fiber membrane was 211 mg/m². The proportion of the number of flat fibers in all hollow fibers was calculated to be 2% by the above method. More specifically, in all hollow fibers, flat fibers having a flatness ratio of 0 to less than 0.2 were 0%, flat fibers having a flatness ratio of 0.2 to less than 0.3 were 0.4%, flat fibers having a flatness ratio of 0.3 to less than 0.4 were 0.5%, and flat fibers having a flatness ratio of 0.4 to 0.5 were 1.1%. Note that the proportion of the number of irregular fibers in all hollow fibers was 0.2%.

### [Example 6]

The hollow fiber membrane blood purifier of Example 6 was obtained in the same manner as in Example 1, except that the pressure difference between the inside (B side) and the outside (D side) of the hollow fiber (inside - outside) was changed to 1.2 MPa in the dewatering step, as shown in Table 1. In this case, the amount of vitamin E immobilized in the hollow fiber membrane was 240 mg/m². The proportion of the number of flat fibers in all hollow fibers was calculated to be 3% by the above method. More specifically, in all hollow fibers, flat fibers having a flatness ratio of 0 to less than 0.2 were 0%, flat fibers having a flatness ratio of 0.2 to less than 0.3 were 0%, flat fibers having a flatness ratio of 0.3 to less than 0.4 were 1.5%, and flat fibers having a flatness ratio of 0.4 to 0.5 were 1.5%. Note that the proportion of the number of irregular fibers in all hollow fibers was 0.2%.

### [Example 7]

The hollow fiber membrane blood purifier of Example 7 was obtained in the same manner as in Example 1, except that vitamin E dissolved in the vitamin E coating solution was changed to 0.004% by mass in the liquid-infusing step, as shown in Table 1. In this case, the amount of vitamin E immobilized in the hollow fiber membrane was 1 mg/m². The proportion of the number of flat fibers in all hollow fibers was calculated to be 1% by the above method. More specifically, in all hollow fibers, flat fibers having a flatness ratio of 0 to less than 0.2 were 0%, flat fibers having a flatness ratio of 0.2 to less than 0.3 were 0.3%, flat fibers having a flatness ratio of 0.3 to less than 0.4 were 0.4%, and flat fibers having a flatness ratio of 0.4 to 0.5 were 0.3%. Note that the proportion of the number of irregular fibers in all hollow fibers was 0%.

### [Example 8]

The hollow fiber membrane blood purifier of Example 8 was obtained in the same manner as in Example 1, except that vitamin E dissolved in the vitamin E coating solution was changed to 0.04% by mass in the liquid-infusing step, as shown in Table 1. In this case, the amount of vitamin E immobilized in the hollow fiber membrane was 10 mg/m². The proportion of the number of flat fibers in all hollow fibers was calculated to be 1% by the above method. More specifically, in all hollow fibers, flat fibers having a flatness ratio of 0 to less than 0.2 were 0%, flat fibers having a flatness ratio of 0.2 to less than 0.3 were 0.2%, flat fibers having a flatness ratio of 0.3 to less than 0.4 were 0.5%, and flat fibers having a flatness ratio of 0.4 to 0.5 were 0.3%. Note that the proportion of the number of irregular fibers in all hollow fibers was 0.3%.

### [Example 9]

The hollow fiber membrane blood purifier of Example 9 was obtained in the same manner as in Example 1, except that the inner diameter of the hollow fiber membrane was changed to 185 µm and the membrane thickness was changed to 45 µm, as shown in Table 1. In this case, the amount of vitamin E immobilized in the hollow fiber membrane was 228 mg/m². The proportion of the number of flat fibers in all hollow fibers was calculated to be 3% by the above method. More specifically, in all hollow fibers, flat fibers having a flatness ratio of 0 to less than 0.2 were 0%, flat fibers having a flatness ratio of 0.2 to less than 0.3 were 0.5%, flat fibers having a flatness ratio of 0.3 to less than 0.4 were 1.2%, and flat fibers having a flatness ratio of 0.4 to 0.5 were 1.3%. Note that the proportion of the number of irregular fibers in all hollow fibers was 0.2%.

### [Comparative Example 1]

The hollow fiber membrane blood purifier of Comparative Example 1 was obtained in the same manner as in Example 9, except that vitamin E dissolved in the vitamin E coating solution was changed to 0.6% by mass in the liquid-infusing step, the pressure difference between the inside (B side) and the outside (D side) of the hollow fiber (inside - outside) was changed to -0.25 MPa in the dewatering step, and the DMAc concentration in the hollow inner liquid was changed to 56% by mass and the spinning rate was changed to 32 m/min upon producing the hollow fiber membrane, as shown in Table 1. In this case, the amount of vitamin E immobilized in the hollow fiber membrane was 141 mg/m². The proportion of the number of flat fibers in all hollow fibers was calculated to be 13% by the above method. More specifically, in all hollow fibers, flat fibers having a flatness ratio of 0 to less than 0.2 were 0%, flat fibers having a flatness ratio of 0.2 to less than 0.3 were 1.5%, flat fibers having a flatness ratio of 0.3 to less than 0.4 were 5.4%, and flat fibers having a flatness ratio of 0.4 to 0.5 were 6.1%. Note that the proportion of the number of irregular fibers in all hollow fibers was 1.2%.

### [Comparative Example 2]

The hollow fiber membrane blood purifier of Comparative Example 2 was obtained in the same manner as in Example 1, except that vitamin E dissolved in the vitamin E coating solution was changed to 0.6% by mass in the liquid-infusing step, the pressure difference between the inside (B side) and the outside (D side) of the hollow fiber (inside - outside) was changed to -0.25 MPa in the dewatering step, and the air flow rate on the B side was changed to 360 mL/min and the air flow rate on the D side was changed to 60 mL/min in the drying step, as shown in Table 1. In this case, the amount of vitamin E immobilized in the hollow fiber membrane was 157 mg/m². The proportion of the number of flat fibers in all hollow fibers was calculated to be 23% by the above method. More specifically, in all hollow fibers, flat fibers having a flatness ratio of 0 to less than 0.2 were 0%, flat fibers having a flatness ratio of 0.2 to less than 0.3 were 2.6%, flat fibers having a flatness ratio of 0.3 to less than 0.4 were 13.4%, and flat fibers having a flatness ratio of 0.4 to 0.5 were 7.0%. Note that the proportion of the number of irregular fibers in all hollow fibers was 3.2%.

### [Comparative Example 3]

The hollow fiber membrane blood purifier of Comparative Example 3 was obtained in the same manner as in Example 1, except that vitamin E dissolved in the vitamin E coating solution was changed to 0.7% by mass in the liquid-infusing step, the pressure difference between the inside (B side) and the outside (D side) of the hollow fiber (inside - outside) was changed to -0.15 MPa in the dewatering step, and the air flow rate on the B side was changed to 420 mL/min and the air flow rate on the D side was changed to 70 mL/min in the drying step, as shown in Table 1. In this case, the amount of vitamin E immobilized in the hollow fiber membrane was 183 mg/m². The proportion of the number of flat fibers in all hollow fibers was calculated to be 10% by the above method. More specifically, in all hollow fibers, flat fibers having a flatness ratio of 0 to less than 0.2 were 0%, flat fibers having a flatness ratio of 0.2 to less than 0.3 were 0.8%, flat fibers having a flatness ratio of 0.3 to less than 0.4 were 6.3%, and flat fibers having a flatness ratio of 0.4 to 0.5 were 2.9%. Note that the proportion of the number of irregular fibers in all hollow fibers was 1.1%.

### [Reference Example 1]

The hollow fiber membrane blood purifier of Reference Example 1 was obtained in the same manner as in Example 1, except that the pressure difference between the inside (B side) and the outside (D side) of the hollow fiber (inside - outside) was changed to 1.5 MPa in the dewatering step, as shown in Table 1. In this case, the amount of vitamin E immobilized in the hollow fiber membrane was 235 mg/m². However, in this production conditions, the hollow fiber was burst in the dewatering step, so that the proportion of the number of flat fibers in all hollow fibers and the leakage time were not measurable.

With respect to the hollow fiber membrane blood purifiers obtained in Examples and Comparative Examples, the leakage time was measured based on the above method. The obtained results were shown in Table 1.

**[Table 1]**

| | | | Example | | | | | | | | | Comparative Example | | | Reference Example |
|---|---|---|---|---|---|---|---|---|---|---|---|---|---|---|---|
| | | | 1 | 2 | 3 | 4 | 5 | 6 | 7 | 8 | 9 | 1 | 2 | 3 | 1 |
| Inner diameter/membrane thickness of hollow fiber membrane | | | 200 µm/43 µm | | | | | | | | 185 µm /45 µm | 185 µm /45 µm | 200 µm/43 µm | | |
| Fat-soluble material immobilization step | Liquid-infusing step | Amount of coating solution | 500 mL | 800 mL | 500 mL | 500 mL | 500 mL | 500 mL | 500 mL | 500 mL | 500 mL | 500 mL | 500 mL | 500 mL | 500 mL |
| | | Coating solution Vitamin E concentration | 0.9wt% | 0.9wt% | 0.9wt% | 0.8wt% | 0.8wt% | 0.9wt% | 0.004wt% | 0.04wt% | 0.9wt% | 0.6wt% | 0.6wt% | 0.7wt% | 0.9wt% |
| | Dewatering step | pressure difference (B side - D side) | -0.05 MPa | -0.05 MPa | 0.05 MPa | -0.05 MPa | 0 MPa | 1.2 MPa | -0.05 MPa | -0.05 MPa | -0.05 MPa | -0.25 MPa | -0.25 MPa | -0.15 MPa | 1.5 MPa |
| | Drying step | Air flow rate (upper stage: B side, lower stage: D side) | 300 mL/min 50 mL/min | 300 mL/min 50 mL/min | 300 mL/min 50 mL/min | 600 mL/min 100 mL/min | 300 mL/min 50 mL/min | 300 mL/min 50 mL/min | 300 mL/min 50 mL/min | 300 mL/min 50 mL/min | 300 mL/min 50 mL/min | 300 mL/min 50 mL/min | 360 mL/min 60 mL/min | 420 mL/min 70 mL/min | 300 mL/min 50 mL/min |
| Proportion of number of flat fibers having flatness ratio of 0.5 or less in all hollow fibers | | | 3% | 4% | 1% | 3% | 2% | 3% | 1% | 1% | 3% | 13% | 23% | 10% | - |
| Breakdown of flat fibers in all hollow fibers | | Flat fiber (flatness ratio: 0 to less than 0.2) | 0% | 0% | 0% | 0% | 0% | 0% | 0% | 0% | 0% | 0% | 0% | 0% | - |
| | | Flat fiber (flatness ratio: 0.2 to less than 0.3) | 0.4% | 0.8% | 0.2% | 0.9% | 0.4% | 0% | 0.3% | 0.2% | 0.5% | 1.5% | 2.6% | 0.8% | - |
| | | Flat fiber (flatness ratio: 0.3 to less than 0.4) | 1.7% | 2.0% | 0.4% | 1.1% | 0.5% | 1.5% | 0.4% | 0.5% | 1.2% | 5.4% | 13.4% | 6.3% | - |
| | | Flat fiber (flatness ratio: 0.4 to less than 0.5) | 0.9% | 1.2% | 0.4% | 1.0% | 1.1% | 1.5% | 0.3% | 0.3% | 1.3% | 6.1% | 7.0% | 2.9% | - |
| Amount of vitamin E immobilized | | | 235 mg/m² | 245 mg/m² | 237 mg/m² | 205 mg/m² | 211 mg/m² | 240 mg/m² | 1 mg/m² | 10 mg/m² | 228 mg/m² | 141 mg/m² | 157 mg/m² | 183 mg/m² | 235 mg/m² |
| Leakage time | | | ≥ 300 min | ≥ 300 min | ≥ 300 min | ≥ 300 min | ≥ 300 min | ≥ 300 min | ≥ 300 min | ≥ 300 min | ≥ 300 min | 210 min | 120 min | 210 min | - |

It was found that the hollow fiber membrane blood purifiers of Examples 1 to 9 can suppress the occurrence of leakage for 300 min or more by controlling the proportion of the number of flat fibers in all hollow fibers to 5% or less.

The present application is based on Japanese Patent application (Japanese Patent Application No. 2021-181881) filed on November 8, 2021, and the content of which is incorporated herein by reference in its entirety.

### Industrial Applicability

According to the present invention, the occurrence of leakage can be suppressed for a long time in the hollow fiber membrane blood purifier containing a fat-soluble material. The hollow fiber membrane blood purifier of the present invention has industrial applicability in, for example, blood purification therapy.

## Claims

1. A hollow fiber membrane blood purifier comprising a hollow fiber membrane and a container to be filled with the hollow fiber membrane, wherein
the hollow fiber membrane in the hollow fiber membrane blood purifier contains a fat-soluble material,
a proportion of the number of flat fibers in all hollow fibers contained in the hollow fiber membrane blood purifier is 5% or less, and the flat fibers have a flatness ratio calculated from the expression (1) of 0.5 or less, and
an amount of the fat-soluble material immobilized in the hollow fiber membrane is 1 to 500 mg/m²,
minor axis of hollow fiber cross section outer diameter/major axis of hollow fiber cross section outer diameter ... expression (1).

2. The hollow fiber membrane blood purifier according to claim 1, wherein the flat fibers having a flatness ratio calculated from the expression (1) of 0.5 or less include flat fibers having a flatness ratio calculated from the expression (1) of 0.2 or more and 0.5 or less.

3. The hollow fiber membrane blood purifier according to claim 1, wherein the flat fibers having a flatness ratio calculated from the expression (1) of 0.5 or less include flat fibers having a flatness ratio calculated from the expression (1) of 0.3 or more and 0.5 or less.

4. The hollow fiber membrane blood purifier according to any one of claims 1 to 3, wherein a substance constituting the hollow fiber membrane contains at least one selected from the group consisting of a hydrophilic polymer and a hydrophobic polymer.

5. The hollow fiber membrane blood purifier according to claim 4, wherein the hydrophilic polymer contains at least one selected from the group consisting of polyvinylpyrrolidone (PVP) and polyethylene glycol (PEG).

6. The hollow fiber membrane blood purifier according to any one of claims 1 to 3, wherein the fat-soluble material contains at least one selected from the group consisting of vitamin A, vitamin D, vitamin E, and vitamin K.

7. The hollow fiber membrane blood purifier according to any one of claims 1 to 3, wherein the amount of the fat-soluble material immobilized in the hollow fiber membrane is 1 to 300 mg/m².

8. The hollow fiber membrane blood purifier according to any one of claims 1 to 3, wherein the amount of the fat-soluble material immobilized in the hollow fiber membrane is 1 to 10 mg/m².

9. The hollow fiber membrane blood purifier according to any one of claims 1 to 3, wherein an inner diameter of the hollow fiber membrane is 170 µm or more and 250 µm or less.

10. The hollow fiber membrane blood purifier according to any one of claims 1 to 3, wherein an inner diameter of the hollow fiber membrane is 180 µm or more and 250 µm or less.

11. The hollow fiber membrane blood purifier according to any one of claims 1 to 3, wherein an inner diameter of the hollow fiber membrane is 180 µm or more 220 µm or less.

12. The hollow fiber membrane blood purifier according to any one of claims 1 to 3, wherein a membrane thickness of the hollow fiber membrane is 10 µm or more and 50 µm or less.
